# EUROPEAN PATENT SPECIFICATION

(11) **EP 3 182 983 B1**
(45) Date of publication and mention of the grant of the patent: **23.10.2019**
(21) Application number: 15833798.0
(22) Date of filing: 20.08.2015
(51) Int. Cl.: A61P 7/02, A61K 35/12, A61K 35/15, C07K 16/44, G01N 33/84, A61K 39/00, A61P 37/06

(54) **ANTIBODIES TO POLYPHOSPHATE DECREASE CLOT FORMATION, DECREASE INFLAMMATION, AND IMPROVE SURVIVAL**
ANTIKÖRPER GEGEN POLYPHOSPHAT ZUR VERRINGERUNG VON GERINNSELBILDUNG, VERRINGERUNG VON ENTZÜNDUNGEN UND ÜBERLEBENSVERBESSERUNG
ANTICORPS ANTI-POLYPHOSPHATES POUR RÉDUIRE LA FORMATION DE CAILLOTS, ATTÉNUER L'INFLAMMATION, ET AMÉLIORER LA SURVIE

(30) Priority: 21.08.2014 US 201462040002 P
(43) Date of publication of application: 28.06.2017
(73) Proprietor: Oklahoma Medical Research Foundation, Oklahoma City, OK 73104 (US); The Board of Trustees of the University of Illinois, Urbana, IL 61801 (US)
(72) Inventor: ESMON, Charles, T., Oklahoma City, OK 73111 (US); ESMON, Naomi, L., Oklahoma City, OK 73111 (US); MORRISSEY, James, H., Champaign, IL 61822 (US)
(74) Representative: Rückerl, Florian
(86) International application number: PCT/US2015/046087
(87) International publication number: WO 2016/029001

(56) References cited:
- US-A1- 2005 169 911
- S. A. SMITH ET AL: "Inhibition of polyphosphate as a novel strategy for preventing thrombosis and inflammation", BLOOD, vol. 120, no. 26, 20 December 2012 (2012-12-20), pages 5103-5110, XP055222894, US ISSN: 0006-4971, DOI: 10.1182/blood-2012-07-444935
- James Morrissey: "Polyphosphate: A Novel Modulator of Hemostasis and Thrombosis | Circulation", Circulation, 26 November 2013 (2013-11-26), XP55444427, Retrieved from the Internet: URL:http://circ.ahajournals.org/content/12 8/Suppl_22/A613 [retrieved on 2018-01-25]
- SMITH STEPHANIE A ET AL: "Polyphosphate modulates blood coagulation and fibrinolysis", PROCEEDINGS NATIONAL ACADEMY OF SCIENCES PNAS, NATIONAL ACADEMY OF SCIENCES, US, vol. 103, no. 4, 24 January 2006 (2006-01-24), pages 903-908, XP002391409, ISSN: 0027-8424, DOI: 10.1073/PNAS.0507195103
- M. LARSSON ET AL: "A Factor XIIa Inhibitory Antibody Provides Thromboprotection in Extracorporeal Circulation Without Increasing Bleeding Risk", SCIENCE TRANSLATIONAL MEDICINE, vol. 6, no. 222, 5 February 2014 (2014-02-05), pages 222ra17-222ra17, XP055303769, US ISSN: 1946-6234, DOI: 10.1126/scitranslmed.3006804
- Felicitas Müller: "Analysis of the factor XII-driven contact system activation in vivo" In: "Doctoral thesis for a doctoral degree at the Graduate School of Life Sciences, Julius-Maximilians-Universität Würzburg, Section Biomedicine submitted by Felicitas Müller from Würzburg", 1 March 2010 (2010-03-01), XP055393815, pages 1-83, * page 36 - page 60 *
- Netgen: "Traitement du choc septique par épuration extracorporelle de l'endotoxine circulante - Revue Médicale Suisse", , 11 December 2013 (2013-12-11), XP055441959, Retrieved from the Internet: URL:https://www.revmed.ch/RMS/2013/RMS-N-4 10/Traitement-du-choc-septique-par-epurati on-extracorporelle-de-l-endotoxine-circula nte [retrieved on 2018-01-18]
- J. H. MORRISSEY ET AL: "Polyphosphate: an ancient molecule that links platelets, coagulation, and inflammation", BLOOD, vol. 119, no. 25, 21 June 2012 (2012-06-21), pages 5972-5979, XP055275863, US ISSN: 0006-4971, DOI: 10.1182/blood-2012-03-306605
- J. M. WAT ET AL: "Polyphosphate suppresses complement via the terminal pathway", BLOOD, vol. 123, no. 5, 30 January 2014 (2014-01-30), pages 768-776, XP055444386, US ISSN: 0006-4971, DOI: 10.1182/blood-2013-07-515726
- SMITH, SA ET AL.: 'Inhibition of polyphosphate as a novel strategy for preventing thrombosis and inflammation.' BLOOD vol. 120, no. 26, 20 December 2012, pages 5103 - 5110, XP055222894
- SEMERARO, N ET AL.: 'Sepsis-associated disseminated intravascular coagulation and thromboembolic disease.' MEDITERR J HEMATOL INFECT DIS. vol. 2, no. 3, 01 January 2010, pages 1 - 18, XP055407511 DOI: 10.4084/MJHID.2010.024
- MULLER, F ET AL.: 'Platelet polyphosphates are proinflammatory and procoagulant mediators in vivo.' CELL vol. 139, no. 6, 11 December 2009, pages 1143 - 1156, XP055407521 DOI: 10.1016/J.CELL.2009.11.001

## Description

### I. Field

The present disclosure relates to the field of medicine, disease and biology. In particular, it relates to the identification and targeting of polyphosphates (polyP) in hyperinflammatory and hypercoagulable disease states.

### II. Related Art

Hyper-inflammatory responses to infection contribute to sepsis. The current understanding of pathogenesis of sepsis is that release of pro-inflammatory cytokines by host cells in response to the invading pathogens causes tissue injury and lethality. Tumor necrosis factor (TNF) and interleukin 1β (IL-10) from macrophages stimulated by lipopolysaccharide (LPS) were identified as early mediators and high mobility group box-1 protein (HMGB1) was identified as a late mediator. Although inhibiting these mediators is protective in animal models, clinical trials using TNF and IL-1β as therapeutic targets in sepsis failed (Wang *et al.,* 1999). Although a potential therapeutic target in septic patients, recent studies indicate that HMGB1 is a weak pro-inflammatory cytokine and levels of HMGB1 correlated only weakly to other pro-inflammatory markers in patients with suspected community-acquired infections and sepsis (Rouhiainen *et al.,* 2007; Gaini *et al.,* 2007).

Hyper-inflammatory responses can lead to a variety of diseases including sepsis. It has recently been shown that extracellular histones released in response to an inflammatory challenge are mediators contributing to endothelial dysfunction, organ failure and death during sepsis. Targeting of similar inflammatory mediators presents a unique therapeutic option for treating these diseases.

S.A. Smith et al. (Blood, 2012, 120(26):5103-5110) reported about the inhibition of polyphosphate as a novel strategy for preventing thrombosis and inflammation. The authors identified a panel of polyP inhibitors including cationic proteins, polymers, and small molecules and reported their effectiveness as anticoagulants in vitro and as antithrombotic and anti-inflammatory agents in vivo using mouse models.

James Morrissey (Summaries of the AHA Council Lectures, 2013, Circulation, Volume 128, Issue Supplement 22) mentions in 2013 polyphosphate as an interesting therapeutic and diagnostic target, including the use of polyphosphates in novel hemostatic agents, as well as the use of inhibitors of polyphosphate function as a novel approach to antithrombotic/anti-inflammatory therapy.

Smith et al. (PNAS, 2006, 103(4):903-908) reported that polyphosphate modulates blood coagulation and fibrinolysis and the authors proposed that polyphosphate released from platelets or microorganisms initially promotes clot formation and stability; subsequent degradation of polyphosphate by blood phosphatases fosters then inhibition of clotting and activation of fibrinolysis during wound healing.

M. Larrson et al. (Sci Transl Med. 2014 Feb 5;6(222):222ra17) reported an anticoagulation therapy based on a plasma protease factor XII function-neutralizing antibody.

F. Müller provided a doctoral thesis ("Analysis of the factor XII-driven contact system activation in vivo" Julius-Maximilians-Universität Würzburg, 1 March 2010) which discloses that activated platelets release inorganic polyP and that targeting polyP with phosphatases interfered with procoagulant activity of activated platelets and blocked platelet - induced thrombosis in mice.

Morrissey et al. (Blood, 2012, 119(25):5972-5979) reviewed the roles of polyP in blood clotting and inflammation and its involvement at the beginning, middle, and end of the blood clotting cascade with prohemostatic, prothrombotic, and proinflammatory effects.

### SUMMARY

In accordance with the disclosure, there is provided an anti-polyphosphate antibody for use in a method of treatment of a medical condition involving extracellular polyphosphate toxicity in a subject. The method includes administering to the subject an anti-polyphosphate antibody *in vivo.* The method may further include administering to the subject an anti-histone antibody. One may also measure polyP level for diagnosing, prognosticating, or assessing treatment in a disease of a subject by measuring a level of polyP. Also provided is a medical device, comprising an anti-polyphosphate antibody, that treats extracorporeal blood flow to reduce polyP load on the subject.

The subject may be a human, dog, cat, horse, monkey, mouse, rat, rabbit, sheep, goat, cow or pig. The disease may be bacterial sepsis, fungal sepsis, virus, Ebola, surgery, traumatic hemorrhage and/or tissue damage, acute pancreatitis, acute respiratory distress syndrome, ischemia-reperfusion injury, cardiovascular disease, cancer, autoimmune disease, chemotherapy toxicity, radiotherapy toxicity, cytokine therapy toxicity, or burn. The subject may suffer from chronic cardiovascular disease, such as atherosclerosis, or tumor angiogenesis or trauma.

In yet another embodiment, there is provided a method of determining a subject's disease prognosis comprising determining the extracellular polyP content of a serum or plasma sample obtained from said subject previously, wherein the presence of extracellular polyP in the sample indicates that the subject is at risk of disease progression. The determining may comprise ELISA or Western blotting using anti-polyP antibodies.

The use of the word "a" or "an" when used in conjunction with the term "comprising" in the claims and/or the specification may mean "one," but it is also consistent with the meaning of "one or more," "at least one," and "one or more than one."

It is contemplated that any embodiment discussed herein can be implemented with respect to any method or composition of the disclosure, and vice versa. Furthermore, compositions and kits of the disclosure can be used to achieve methods of the disclosure.

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

### BRIEF DESCRIPTION OF THE DRAWINGS

The following drawings form part of the present specification and are included to further demonstrate certain aspects of the present disclosure. The disclosure may be better understood by reference to one or more of these drawings in combination with the detailed description of specific embodiments presented herein.
**FIG. 1** shows structure of inorganic polyP.
**FIG. 2** demonstrates time to clot differences in 5 different trials, 4 of which have polyP, demonstrating that with sufficient concentration of anti-polyP antibodies (e.g., PP2055) that polyP effect on time to clot is almost neutralized.
**FIG. 3** shows increased survival in mice administered anti-polyP antibodies and lipopolysaccharide v. control mice that were administered lipopolysaccharide without anti-polyP antibodies, demonstrating that anti-polyP antibodies protect from death in the acute inflammatory model.
**FIG. 4** shows that thrombin-antithrombin complex (TAT) levels are lower in mice administered anti-polyP antibodies and lipopolysaccharide v. control mice that were administered lipopolysaccharide without anti-phosphate antibodies, demonstrating that anti-polyP antibodies strongly attenuate the coagulopathy induced by lipopolysaccharide.
**FIG. 5** shows the results of coagulation assays in which coagulation was initiated by recalcification of citrated human plasma in the presence of polyP.
**FIG. 6** shows the results of coagulation assays in which coagulation was initiated by recalcification of citrated human plasma in the presence of RNA (polyguanylic acid).

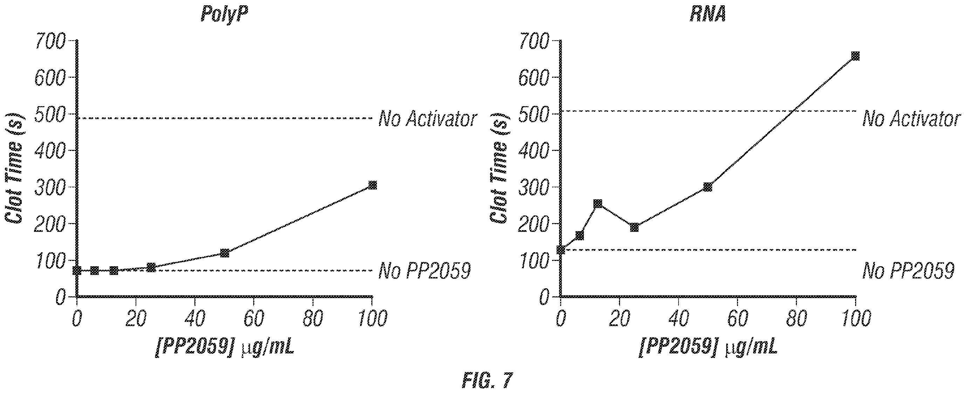

**FIG. 7** shows PP2059 Inhibition of polyP and RNA (polyguanylic acid, polyG) mediated contact activation in human plasma. 25 µL of PP2059 (final concentrations listed above) and either 25 µL 10 µg/mL (final concentration) long-chain polyP or 25 µg/mL (final concentration) polyG were pre-incubated for 10 minutes at 37 °C, then incubated with 50 µL pooled normal human plasma for 3 minutes before the initiation of clotting by the addition of 50 µL of 25 mM calcium chloride. Final concentration for buffer components were 41.7 mM imidazole pH 8.0, 25 µM 80:20 phosphatidylcholine: phosphatidylserine liposomes, 33% pooled normal human plasma, 8.3 mM Ca²⁺.

### DETAILED DESCRIPTION

As discussed above, hyper-inflammatory responses can lead to a variety of diseases including sepsis. Inorganic polyphosphate (polyP) is a linear chain of phosphate residues that is found in many organs, and polyP is released from dense granules in platelets and is also secreted by activated mast cells. PolyP activates the intrinsic pathway of coagulation that also induces inflammation. It is highly desirable to identify inhibitors to polyP that would not be toxic, which would allow investigation of the roles of polyP in disease, and which would lead to diagnostic and therapeutic methods and apparatuses.

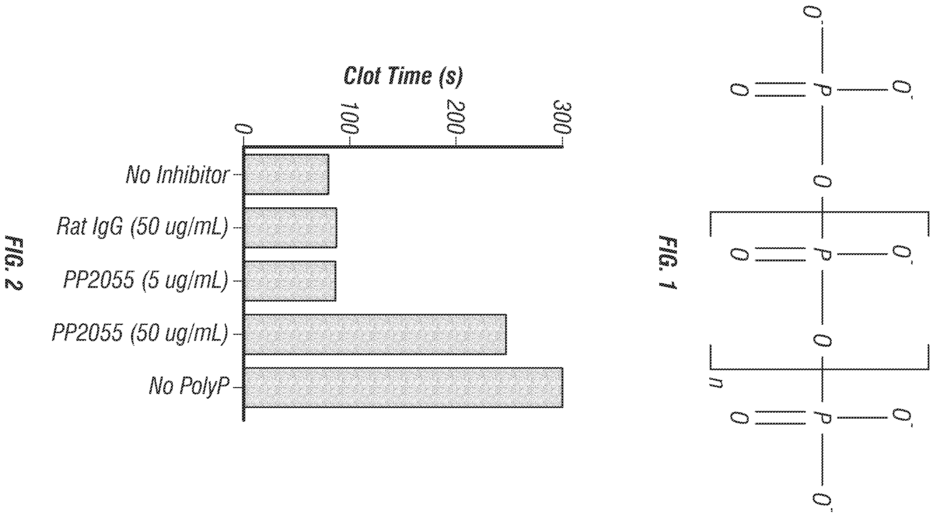

One candidate for an inhibitor of polyP would be an antibody. The ubiquitous nature of polyP raised the concern that it may not be possible for an animal to mount an immune response against this molecule, owing to immune tolerance. In addition, the simple, repeating structure of polyP raised concerns about the feasibility of generating an antibody against it, as such simple molecules may not be antigenic. And finally, it was very unclear that antibodies to polyP, even if they could be generated, would be able to effectively block the procoagulant activity of polyP. See FIG. 1. However, the inventors have demonstrated the feasibility of generating neutralizing anti-polyP antibodies that should lead to positive diagnostic and therapeutic results.

Thus, in accordance some embodiments, there is provided an anti-polyphosphate antibody for use in a method of treatment of a medical condition involving extracellular polyP toxicity in a subject comprising administering to the subject an anti-polyP antibody, optionally with an anti-histone antibody. One may also measure a level of polyP in a serum or plasma sample obtained from a subject for diagnosis, prognosis, and/or treatment response quantification. In addition, one may employ an apparatus comprising an anti-polyphosphate antibody to remove polyP, such as by way of example and not limitation, extracorporeal removal in a heart bypass machine. These and other aspects of the disclosure are described in greater detail below.

### I. Hyper-Inflammatory Disease States

The present disclosure contemplates diagnosing and intervening in a variety of disease states that involve the release of polyPs and the resulting extracellular toxicity therefrom. A number of these disease states are described below.

### A. Sepsis

Sepsis is a serious medical condition characterized by a whole-body inflammatory state caused by infection. Traditionally the term sepsis has been used interchangeably with septicaemia and septicemia ("blood poisoning"). However, these terms are no longer considered synonymous; septicemia is considered a subset of sepsis.

Symptoms of sepsis are often related to the underlying infectious process. When the infection crosses into sepsis, the resulting symptoms are that of systemic inflammatory response syndrome (SIRS): general inflammation, fever, elevated white blood cell count (leukocytosis), and raised heart rate (tachycardia) and breathing rate (tachypnea). Secondary to the above, symptoms also include flu like chills.

The immunological response that causes sepsis is a systemic inflammatory response causing widespread activation of inflammation and coagulation pathways. This may progress to dysfunction of the circulatory system and, even under optimal treatment, may result in the multiple organ dysfunction syndrome and eventually death.

Sepsis is considered present if infection is highly suspected or proven and two or more of the following systemic inflammatory response syndrome (SIRS) criteria are met: heart rate >90 beats per minute body temperature <36 °C (96.8 °F) or >38 °C. (100.4 °F) hyperventilation (high respiratory rate) >20 breaths per minute or, on blood gas, a PₐCO₂ less than 32 mm Hg white blood cell count <4000 cells/mm³ or >12000 cells/mm³ (<4 × 10⁹ or >12 × 10⁹ cells/L), or greater than 10% band forms (immature white blood cells). Consensus definitions however continue to evolve with the latest expanding the list of signs and symptoms of sepsis to reflect clinical bedside experience.

The more critical subsets of sepsis are severe sepsis (sepsis with acute organ dysfunction) and septic shock (sepsis with refractory arterial hypotension). Alternatively, when two or more of the systemic inflammatory response syndrome criteria are met without evidence of infection, patients may be diagnosed simply with "SIRS." Patients with SIRS and acute organ dysfunction may be termed "severe SIRS."

Patients are defined as having "severe sepsis" if they have sepsis plus signs of systemic hypoperfusion; either end organ dysfunction or a serum lactate greater than 4 mmol/dL. Patients are defined as having septic shock if they have sepsis plus hypotension after an appropriate fluid bolus (typically 20 ml/kg of crystalloid). The criteria for diagnosing an adult with sepsis do not apply to infants under one month of age. In infants, only the presence of infection plus a "constellation" of signs and symptoms consistent with the systemic response to infection are required for diagnosis.

The therapy of sepsis rests on antibiotics, surgical drainage of infected fluid collections, fluid replacement and appropriate support for organ dysfunction. This may include hemodialysis in kidney failure, mechanical ventilation in pulmonary dysfunction, transfusion of blood products, and drug and fluid therapy for circulatory failure. Ensuring adequate nutrition, if necessary by parenteral nutrition, is important during prolonged illness.

A problem in the adequate management of septic patients has been the delay in administering therapy after sepsis has been recognized. Published studies have demonstrated that for every hour delay in the administration of appropriate antibiotic therapy there is an associated 7% rise in mortality. A large international collaboration was established to educate people about sepsis and to improve patient outcomes with sepsis, entitled the "Surviving Sepsis Campaign." The Campaign has published an evidence-based review of management strategies for severe sepsis, with the aim to publish a complete set of guidelines in subsequent years.

Standard treatment of infants with suspected sepsis consists of supportive care, maintaining fluid status with intravenous fluids, and the combination of a beta-lactam antibiotic (such as ampicillin) with an aminoglycoside such as gentamicin.

### B. Trauma

Physical trauma is a serious and body-altering physical injury, such as the removal of a limb. Blunt force trauma is a type of physical trauma caused by impact or other force applied from or with a blunt object, whereas penetrating trauma is a type of physical trauma in which the skin or tissues are pierced by an object. Trauma can also be described as both unplanned, such as an accident, or planned, in the case of surgery. Both can be characterized by mild to severe tissue damage, blood loss and/or shock, and both may lead to subsequent infection, including sepsis. The present disclosure provides for treatment of trauma, including both pre-treatment (in the case of a medical procedure) and treatment after trauma injury as occurred.

### i. Surgery

Surgery uses operative manual and instrumental techniques on a patient to investigate and/or treat a pathological condition such as disease or injury, to help improve bodily function or appearance, or sometimes for some other reason. The present disclosure can address trauma resulting from surgeries, as defined further below.

As a general rule, a procedure is considered surgical when it involves cutting of a patient's tissues or closure of a previously sustained wound. Other procedures that do not necessarily fall under this rubric, such as angioplasty or endoscopy, may be considered surgery if they involve common surgical procedure or settings, such as use of a sterile environment, anesthesia, antiseptic conditions, typical surgical instruments, and suturing or stapling. All forms of surgery are considered invasive procedures; so-called noninvasive surgery usually refers to an excision that does not penetrate the structure being addressed (*e.g.,* laser ablation of the cornea) or to a radiosurgical procedure (*e.g.,* irradiation of a tumor). Surgery can last from minutes to hours.

Surgical procedures are commonly categorized by urgency, type of procedure, body system involved, degree of invasiveness, and special instrumentation. Elective surgery is done to correct a non-life-threatening condition, and is carried out at the patient's request, subject to the surgeon's and the surgical facility's availability. Emergency surgery is surgery that must be done quickly to save life, limb, or functional capacity. Exploratory surgery is performed to aid or confirm a diagnosis. Therapeutic surgery treats a previously diagnosed condition.

Amputation involves cutting off a body part, usually a limb or digit. Replantation involves reattaching a severed body part. Reconstructive surgery involves reconstruction of an injured, mutilated, or deformed part of the body. Cosmetic surgery is done to improve the appearance of an otherwise normal structure. Excision is the cutting out of an organ, tissue, or other body part from the patient. Transplant surgery is the replacement of an organ or body part by insertion of another from different human (or animal) into the patient. Removing an organ or body part from a live human or animal for use in transplant is also a type of surgery.

When surgery is performed on one organ system or structure, it may be classed by the organ, organ system or tissue involved. Examples include cardiac surgery (performed on the heart), gastrointestinal surgery (performed on the digestive tract and its accessory organs), and orthopedic surgery (performed on bones and/or muscles).

Minimally invasive surgery involves smaller outer incision(s) to insert miniaturized instruments within a body cavity or structure, as in laparoscopic surgery or angioplasty. By contrast, an open surgical procedure requires a large incision to access the area of interest. Laser surgery involves use of a laser for cutting tissue instead of a scalpel or similar surgical instruments. Microsurgery involves the use of an operating microscope for the surgeon to see small structures. Robotic surgery makes use of a surgical robot, such as Da Vinci or Zeus surgical systems, to control the instrumentation under the direction of the surgeon.

### ii. Traumatic Hemorrhage

Traumatic hemorrhage accounts for much of the wide ranging international impact of injury, causing a large proportion of deaths and creating great morbidity in the injured. Despite differences in pre-hospital care, the acute management of traumatic hemorrhage is similar around the world and follows well-accepted published guidelines. A critically injured patient's care occurs as three, often overlapping segments: the resuscitative, operative, and critical care phases. The diagnosis and control of bleeding should be a high priority during all of the phases of trauma care and is especially important in the patient who is in hemorrhagic shock. Early attempts at hemorrhage control include direct control of visible sources of severe bleeding with direct pressure, pressure dressings, or tourniquets; stabilization of long bone and pelvic fractures; and keeping the patient warm. During the resuscitative phase, warmed intravenous fluids, hypotensive resuscitation prior to surgical control of hemorrhage, and appropriate transfusion of blood and blood products are provided. In the operative phase, surgical control of the hemorrhage and any other injury, and additional transfusion is provided. Finally, the critical care phase provides for post-operative support and tissue perfusion.

### C. Acute Pancreatitis

Acute pancreatitis is rapidly-onset inflammation of the pancreas. Depending on its severity, it can have severe complications and high mortality despite treatment. While mild cases are often successfully treated with conservative measures or laparoscopy, severe cases require invasive surgery (often more than one intervention) to contain the disease process.

### D. Acute Respiratory Distress Syndrome

Acute respiratory distress syndrome (ARDS), also known as respiratory distress syndrome (RDS) or adult respiratory distress syndrome (in contrast with IRDS) is a serious reaction to various forms of injuries to the lung. This is the most important disorder resulting in increased permeability pulmonary edema.

ARDS is a severe lung disease caused by a variety of direct and indirect insults. It is characterized by inflammation of the lung parenchyma leading to impaired gas exchange with concomitant systemic release of inflammatory mediators causing inflammation, hypoxemia and frequently resulting in multiple organ failure. This condition is life threatening and often lethal, usually requiring mechanical ventilation and admission to an intensive care unit. A less severe form is called acute lung injury (ALI).

ARDS can occur within 24 to 48 hours of an injury or attack of acute illness. In such a case the patient usually presents with shortness of breath, tachypnea, and symptoms related to the underlying cause, *i.e.,* shock. Long-term illnesses can also trigger it, such as malaria. The ARDS may then occur sometime after the onset of a particularly acute case of the infection.

An arterial blood gas analysis and chest X-ray allow formal diagnosis by inference using the aforementioned criteria. Although severe hypoxemia is generally included, the appropriate threshold defining abnormal PaO₂ has never been systematically studied. Any cardiogenic cause of pulmonary edema should be excluded. This can be done by placing a pulmonary artery catheter for measuring the pulmonary artery wedge pressure. However, this is not necessary and is now rarely done as abundant evidence has emerged demonstrating that the use of pulmonary artery catheters does not lead to improved patient outcomes in critical illness including ARDS. Plain chest X-rays are sufficient to document bilateral alveolar infiltrates in the majority of cases. While CT scanning leads to more accurate images of the pulmonary parenchyma in ARDS, it has little utility in the clinical management of patients with ARDS, and remains largely a research tool.

Acute respiratory distress syndrome is usually treated with mechanical ventilation in the Intensive Care Unit. Ventilation is usually delivered through orotracheal intubation, or tracheostomy whenever prolonged ventilation (>2 weeks) is deemed inevitable. The possibilities of non-invasive ventilation are limited to the very early period of the disease or, better, to prevention in individuals at risk for the development of the disease (atypical pneumonias, pulmonary contusion, major surgery patients). Treatment of the underlying cause is imperative, as it tends to maintain the ARDS picture. Appropriate antibiotic therapy must be administered as soon as microbiological culture results are available. Empirical therapy may be appropriate if local microbiological surveillance is efficient. More than 60% ARDS patients experience a (nosocomial) pulmonary infection either before or after the onset of lung injury. The origin of infection, when surgically treatable, must be operated on. When sepsis is diagnosed, appropriate local protocols should be enacted.

### E. Ischemia-Reperfusion Injury

Reperfusion injury refers to damage to tissue caused when blood supply returns to the tissue after a period of ischemia. The absence of oxygen and nutrients from blood creates a condition in which the restoration of circulation results in inflammation and oxidative damage through the induction of oxidative stress rather than restoration of normal function.

The damage of reperfusion injury is due in part to the inflammatory response of damaged tissues. White blood cells carried to the area by the newly returning blood release a host of inflammatory factors such as interleukins as well as free radicals in response to tissue damage. The restored blood flow reintroduces oxygen within cells that damages cellular proteins, DNA, and the plasma membrane. Damage to the cell's membrane may in turn cause the release of more free radicals. Such reactive species may also act indirectly in redox signaling to turn on apoptosis. Leukocytes may also build up in small capillaries, obstructing them and leading to more ischemia.

Reperfusion injury plays a part in the brain's ischemic cascade, which is involved in stroke and brain trauma. Repeated bouts of ischemia and reperfusion injury also are thought to be a factor leading to the formation and failure to heal of chronic wounds such as pressure sores and diabetic foot ulcers. Continuous pressure limits blood supply and causes ischemia, and the inflammation occurs during reperfusion. As this process is repeated, it eventually damages tissue enough to cause a wound.

In prolonged ischemia (60 min or more), hypoxanthine is formed as breakdown product of ATP metabolism. The enzyme xanthine dehydrogenase is converted to xanthine oxidase as a result of the higher availability of oxygen. This oxidation results in molecular oxygen being converted into highly reactive superoxide and hydroxyl radicals. Xanthine oxidase also produces uric acid, which may act as both a prooxidant and as a scavenger of reactive species such as peroxinitrite. Excessive nitric oxide produced during reperfusion reacts with superoxide to produce the potent reactive species peroxynitrite. Such radicals and reactive oxygen species attack cell membrane lipids, proteins, and glycosaminoglycans, causing further damage. They may also initiate specific biological processes by redox signaling.

### F. Cardiovascular Disease

Cardiovascular disease refers to the class of diseases that involve the heart or blood vessels (arteries and veins). While the term technically refers to any disease that affects the cardiovascular system, it is usually used to refer to those related to atherosclerosis (arterial disease). These conditions have similar causes, mechanisms, and treatments. Treatment of cardiovascular disease depends on the specific form of the disease in each patient, but effective treatment always includes preventive lifestyle changes discussed above. Medications, such as blood pressure reducing medications, aspirin and the statin cholesterol-lowering drugs may be helpful. In some circumstances, surgery or angioplasty may be warranted to reopen, repair, or replace damaged blood vessels.

Most Western countries face high and increasing rates of cardiovascular disease. Each year, heart disease kills more Americans than cancer. Diseases of the heart alone caused 30% of all deaths, with other diseases of the cardiovascular system causing substantial further death and disability. Up until the year 2014, it was the number 1 cause of death and disability in the United States and most European countries. A large histological study (PDAY) showed vascular injury accumulates from adolescence, making primary prevention efforts necessary from childhood.

Some biomarkers are thought to offer a more detailed risk of cardiovascular disease. However, the clinical value of these biomarkers is questionable. Currently, biomarkers which may reflect a higher risk of cardiovascular disease include: higher fibrinogen and PAI-1 blood concentrations elevated homocysteine, or even upper half of normal elevated blood levels of asymmetric dimethylarginine high inflammation as measured by C-reactive protein elevated blood levels of B-type natriuretic peptide (BNP) Various forms of cardiovascular disease include aneurysms, angina, arrhythmia, atherosclerosis, cardiomyopathy, cerebrovascular disease, congenital heart disease, congestive heart failure, myocarditis, valve disease, coronary artery disease, dilated cardiomyopathy, diastolic dysfunction, endocarditis, high blood pressure (hypertension), hypertrophic cardiomyopathy, mitral valve prolapse, myocardial infarction, and venous thromboembolism.

### G. Autoimmune/Inflammatory Disease

The present disclosure contemplates the treatment of a variety of autoimmune and/or inflammatory disease states such as spondyloarthropathy, ankylosing spondylitis, psoriatic arthritis, reactive arthritis, enteropathic arthritis, ulcerative colitis, Crohn's disease, irritable bowel disease, rheumatoid arthritis, juvenile rheumatoid arthritis, familial Mediterranean fever, amyotrophic lateral sclerosis, Sjogren's syndrome, early arthritis, viral arthritis, multiple sclerosis, or psoriasis. The diagnosis and treatment of these diseases are well documented in the literature.

### H. Chemotherapy, Radiotherapy and Cytokine Therapy Toxicity

Various forms of cancer therapy, including chemotherapy, radiation, and cytokines, are associated with toxicity, sometimes severe, in the cancer patient. To the extent that the toxicity is caused at least in part by the extracellular actions of polyPs, the present disclosure seeks to reduce this toxicity using the pharmaceutical compositions of the present disclosure, thereby reducing or alleviating discomfort on the part of the patient, as well as permitting higher doses of the therapy.

### I. Burns

In medicine, a burn may be an injury caused by heat, cold, electricity, chemicals, friction or radiation. First-degree burns are usually limited to redness (erythema), a white plaque, and minor pain at the site of injury. These burns usually extend only into the epidermis. Second-degree burns additionally fill with clear fluid, have superficial blistering of the skin, and can involve more or less pain depending on the level of nerve involvement. Second-degree burns involve the superficial (papillary) dermis and may also involve the deep (reticular) dermis layer. Third-degree burns additionally have charring of the skin, and produce hard, leather-like eschars. An eschar is a scab that has separated from the unaffected part of the body. Frequently, there is also purple fluid. These types of burns are often painless, because nerve endings have been destroyed in the burned areas. Serious burns, especially if they cover large areas of the body, can cause death; any hint of burn injury to the lungs (e.g., through smoke inhalation) is a medical emergency.

Burns that injure the tissues underlying the skin, such as the muscles or bones, are sometimes categorized as fourth-degree burns. These burns are broken down into three additional degrees: fourth-degree burns result in the skin being irretrievably lost, fifth-degree burns result in muscle being irretrievably lost, and sixth-degree burns result in bone being charred.

A newer classification of "Superficial Thickness," "Partial Thickness" (which is divided into superficial and deep categories) and "Full Thickness" relates more precisely to the epidermis, dermis and subcutaneous layers of skin and is used to guide treatment and predict outcome.

Chemical burns are usually caused by chemical compounds, such as sodium hydroxide (lye), silver nitrate, and more serious compounds (such as sulfuric acid). Most chemicals (but not all) that can cause moderate to severe chemical burns are strong acids or bases. Nitric acid, as an oxidizer, is possibly one of the worst burn-causing chemicals. Hydrofluoric acid can eat down to the bone and its burns are often not immediately evident. Most chemicals that can cause moderate to severe chemical burns are called caustic.

Electrical burns are generally symptoms of electric shock, being struck by lightning, being defibrillated or cardioverted without conductive gel, *etc.* The internal injuries sustained may be disproportionate to the size of the "burns" seen--as these are only the entry and exit wounds of the electrical current.

Burns are assessed in terms of total body surface area (TBSA), which is the percentage affected by partial thickness or full thickness burns (superficial thickness burns are not counted). The rule of nines is used as a quick and useful way to estimate the affected TBSA. The first step in managing a person with a burn is to stop the burning process. With dry powder burns, the powder should be brushed off first. With other burns, the affected area should be rinsed with a large amount of clean water to remove foreign bodies and help stop the burning process. Cold water should never be applied to any person with extensive burns, as it may severely compromise the burn victim's temperature status. At this stage of management, it is also critical to assess the airway status. If the patient was involved in a fire, then it must be assumed that he or she has sustained inhalation injury until proven otherwise, and treatment should be managed accordingly.

Once the burning process has been stopped, and airway status is ensured, the patient should be volume resuscitated according to the standard of care for subjects with burns. Inadequate fluid resuscitation causes renal failure and death. Severe edema in full thickness burns may be treated by escharotomy.

### J. Cancer

Cancer and pre-malignant hyperproliferative diseases are now understood to be a disorder having significant inflammatory components. The great majority of cancers, some 90-95% of cases, are due to environmental factors. The remaining 5-10% are due to inherited genetics. "Environmental," as used by cancer researchers, means any cause that is not inherited genetically, such as lifestyle, economic and behavioral factors, and not merely pollution. Common environmental factors that contribute to cancer death include tobacco (25-30%), diet and obesity (30-35%), infections (15-20%), radiation (both ionizing and non-ionizing, up to 10%), stress, lack of physical activity, and environmental pollutants.

One of the hallmarks of cancer is that interruption of the cell's normal apoptotic cycle. In addition, five other characteristics of cancer have been proposed: self-sufficiency in growth signaling, insensitivity to anti-growth signals, enabling of a limitless replicative potential, induction and sustainment of angiogenesis and activation of metastasis and invasion of tissue.

Cancer is fundamentally a disease of tissue growth regulation failure. In order for a normal cell to transform into a cancer cell, the genes that regulate cell growth and differentiation must be altered. The affected genes are divided into two broad categories. Oncogenes are genes that promote cell growth and reproduction. Tumor suppressor genes are genes that inhibit cell division and survival. Malignant transformation can occur through the formation of novel oncogenes, the inappropriate over-expression of normal oncogenes, or by the under-expression or disabling of tumor suppressor genes. Typically, changes in *many* genes are required to transform a normal cell into a cancer cell.

Genetic changes can occur at different levels and by different mechanisms. The gain or loss of an entire chromosome can occur through errors in mitosis. More common are mutations, which are changes in the nucleotide sequence of genomic DNA. Large-scale mutations involve the deletion or gain of a portion of a chromosome. Genomic amplification occurs when a cell gains many copies (often 20 or more) of a small chromosomal locus, usually containing one or more oncogenes and adjacent genetic material. Translocation occurs when two separate chromosomal regions become abnormally fused, often at a characteristic location.

Small-scale mutations include point mutations, deletions, and insertions, which may occur in the promoter region of a gene and affect its expression, or may occur in the gene's coding sequence and alter the function or stability of its protein product. Disruption of a single gene may also result from integration of genomic material from a DNA virus or retrovirus, leading to the expression of *viral* oncogenes in the affected cell and its descendants.

Replication of the enormous amount of data contained within the DNA of living cells will probabilistically result in some errors (mutations). Complex error correction and prevention is built into the process, and safeguards the cell against cancer. If significant error occurs, the damaged cell can "self-destruct" through programmed cell death, termed apoptosis. If the error control processes fail, then the mutations will survive and be passed along to daughter cells.

Cancer cells that may be treated according to the present disclosure include but are not limited to cells from the bladder, blood, bone, bone marrow, brain, breast, colon, esophagus, gastrointestine, gum, head, kidney, liver, lung, nasopharynx, neck, ovary, prostate, skin, stomach, pancreas, testis, tongue, cervix, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; Paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; Mullerian mixed tumor; nephroblastoma; hepatoblastoma; carcinosarcoma; mesenchymoma, malignant; Brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; Kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; Ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia. In certain aspects, the tumor may comprise an osteosarcoma, angiosarcoma, rhabdosarcoma, leiomyosarcoma, Ewing sarcoma, glioblastoma, neuroblastoma, or leukemia.

### II. Polyphosphates

In biology, inorganic polyPs are found in many organs of subjects, including humans. PolyP is released from dense granules in platelets and from certain secretory granules in mast cells. PolyP is accumulated by many infectious microorganisms, and polyP can be critical for cell viability in bacteria. Damaged microorganisms may release polyP, and the released polyP may induce injury on the host subject. PolyP secreted by platelets (60 to 100 phosphate units long) are considerably shorter than the polyP found in infectious microorganisms (ranging up to hundreds of phosphate units long, and frequently in excess of 1000 phosphate units long). Bacterial polyP is very potent at triggering the contact pathway of blood clotting. Short polyP polymers are effective at accelerating certain blood clotting reactions in the common pathway of the plasma clotting cascade. PolyP activates the intrinsic pathway of coagulation that also induces inflammation.

It also is contemplated in the present disclosure that various anti-polyP antibodies may block polyP toxicity. The present disclosure also may employ antibodies that comprise modified, non-natural and/or unusual amino acids. The treatment may occur *in vivo* and can occur extracorporeally. For example, extracellular polyphosphate might be "cleaned" or neutralized with blood flow through a cardiac bypass machine or extracorporeal life-support (*e.g.,* PLS system from Maquet Getinge Group), by way of example and not limitation.

### III. Antibodies and Immunoassays

It will be understood that polyclonal or monoclonal antibodies that bind immunologically to polyPs will have use in several applications. These include diagnostic kits and methods of detecting polyPs, as well as therapeutic intervention. Means for preparing and characterizing antibodies are well known in the art (see, *e.g*., Antibodies: A Laboratory Manual, 1988). The term "antibody" as used herein is used to refer to any antibody-like molecule that has an antigen binding region, and includes antibody fragments such as Fab', Fab, F(ab')₂, single domain antibodies (DAB's), Fv, scFv (single-chain Fv), and the like.

### A. Polyclonal Antisera

Polyclonal antisera are prepared by immunizing an animal with an immunogenic composition in accordance with the present disclosure and collecting antisera from that immunized animal. A wide range of animal species can be used for the production of antisera. Typically the animal used for production of anti-antisera is a rabbit, a mouse, a rat, a hamster, a guinea pig or a goat. Because of the relatively large blood volume of rabbits, a rabbit is a choice for production of polyclonal antibodies.

As is well known in the art, a given composition may vary in its immunogenicity. It is often necessary therefore to boost the host immune system, as may be achieved by coupling a peptide or polypeptide immunogen to a carrier. Exemplary carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include glutaraldehyde, m-maleimidobencoyl-N-hydroxysuccinimide ester, carbodiimide and bis-biazotized benzidine. Means for conjugating polyP covalently to amino groups on a carrier protein using the cross-linking agent, EDAC (1-ethyl-3-[3-dimetyhlamino-propyl]carbodiimide), are described in: S. H. Choi, J. N. R. Collins, S. A. Smith, R. L. Davis-Harrison, C. M. Rienstra and J. H. Morrissey (2010) Phosphoramidate end labeling of inorganic polyphosphates: facile manipulation of polyphosphate for investigating and modulating its biological activities. See Biochemistry 49(45):9935-9941.

As also is well known in the art, the immunogenicity of a particular immunogen composition can be enhanced by the use of non-specific stimulators of the immune response, known as adjuvants. Exemplary adjuvants include complete Freund's adjuvant (a non-specific stimulator of the immune response containing killed Mycobacterium tuberculosis), incomplete Freund's adjuvants and aluminum hydroxide adjuvant.

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization.

A second, booster injection, also may be given. The process of boosting and titering is repeated until a suitable titer is achieved. When a desired level of immunogenicity is obtained, the immunized animal can be bled and the serum isolated and stored, or the animal can be used to generate mAbs (below).

For production of rabbit polyclonal antibodies, the animal can be bled through an ear vein or alternatively by cardiac puncture. The procured blood is allowed to coagulate and then centrifuged to separate serum components from whole cells and blood clots. The serum may be used as is for various applications or else the desired antibody fraction may be purified by well-known methods, such as affinity chromatography using another antibody or a peptide bound to a solid matrix or protein A followed by antigen (peptide) affinity column for purification.

### B. Monoclonal Antibodies

MAbs may be readily prepared through use of well-known techniques, such as those exemplified in U.S. Pat. No. 4,196,265. Typically, this technique involves immunizing a suitable animal with a selected immunogen composition, e.g., a purified or partially purified polyPs, or fragments therefrom. The immunizing composition is administered in a manner effective to stimulate antibody producing cells.

The methods for generating monoclonal antibodies (MAbs) generally begin along the same lines as those for preparing polyclonal antibodies. Rodents such as mice and rats are exemplary animals; however, the use of rabbit, sheep, goat, monkey cells also is possible. The use of rats may provide certain advantages (Goding, 1986, pp. 60-61), but mice may be used.

The animals are injected with antigen, generally as described above. The antigen may be coupled to carrier molecules such as keyhole limpet hemocyanin if necessary. The antigen would typically be mixed with adjuvant, such as Freund's complete or incomplete adjuvant. Booster injections with the same antigen would occur at approximately two-week intervals.

Following immunization, somatic cells with the potential for producing antibodies, specifically B lymphocytes (B cells), are selected for use in the MAb generating protocol. These cells may be obtained from biopsied spleens or lymph nodes. Spleen cells and lymph node cells may be used, the former because they are a rich source of antibody-producing cells that are in the dividing plasmablast stage.

Often, a panel of animals will have been immunized and the spleen of animal with the highest antibody titer will be removed and the spleen lymphocytes obtained by homogenizing the spleen with a syringe. Typically, a spleen from an immunized mouse contains approximately 5×10⁷ to 2×10⁸ lymphocytes.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas).

Any one of a number of myeloma cells may be used, as are known to those of skill in the art (Goding, pp. 65-66, 1986; Campbell, pp. 75-83, 1984). For example, where the immunized animal is a mouse, one may use P3-X63/Ag8, X63-Ag8.653, NS1/1.Ag 4 1, Sp210-Ag14, FO, NSO/U, MPC-11, MPC11-X45-GTG 1.7 and S194/5XX0 Bul; for rats, one may use R210.RCY3, Y3-Ag 1.2.3, IR983F and 4B210; and U-266, GM1500-GRG2, LICR-LON-HMy2 and UC729-6 are all useful in connection with human cell fusions. One particular murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/0 non-producer cell line.

Methods for generating hybrids of antibody-producing spleen or lymph node cells and myeloma cells usually comprise mixing somatic cells with myeloma cells in a 2:1 proportion, though the proportion may vary from about 20:1 to about 1:1, respectively, in the presence of an agent or agents (chemical or electrical) that promote the fusion of cell membranes. Fusion methods using Sendai virus have been described by Kohler and Milstein (1975; 1976), and those using polyethylene glycol (PEG), such as 37% (v/v) PEG, by Gefter *et al.* (1977). The use of electrically induced fusion methods also is appropriate (Goding pp. 71-74, 1986).

Fusion procedures usually produce viable hybrids at low frequencies, about 1×10⁻⁶ to 1×10⁻⁸. However, this does not pose a problem, as the viable, fused hybrids are differentiated from the parental, infused cells (particularly the infused myeloma cells that would normally continue to divide indefinitely) by culturing in a selective medium. The selective medium is generally one that contains an agent that blocks the de novo synthesis of nucleotides in the tissue culture media. Exemplary agents are aminopterin, methotrexate, and azaserine. Aminopterin and methotrexate block de novo synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine.

A particular selection medium is HAT. Only cells capable of operating nucleotide salvage pathways are able to survive in HAT medium. The myeloma cells are defective in key enzymes of the salvage pathway, e.g., hypoxanthine phosphoribosyl transferase (HPRT), and they cannot survive. The B cells can operate this pathway, but they have a limited life span in culture and generally die within about two weeks. Therefore, the only cells that can survive in the selective media are those hybrids formed from myeloma and B cells.

This culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, toxicity assays, plaque assays, dot immunobinding assays, and the like. The selected hybridomas would then be serially diluted and cloned into individual antibody-producing cell lines, which clones can then be propagated indefinitely to provide mAbs. The cell lines may be exploited for mAb production in two basic ways.

A sample of the hybridoma can be injected (often into the peritoneal cavity) into a histocompatible animal of the type that was used to provide the somatic and myeloma cells for the original fusion (e.g., a syngeneic mouse). Optionally, the animals are primed with a hydrocarbon, especially oils such as pristane (tetramethylpentadecane) prior to injection. The injected animal develops tumors secreting the specific monoclonal antibody produced by the fused cell hybrid. The body fluids of the animal, such as serum or ascites fluid, can then be tapped to provide mAbs in high concentration.

The individual cell lines could also be cultured *in vitro,* where the mAbs are naturally secreted into the culture medium from which they can be readily obtained in high concentrations. mAbs produced by either means may be further purified, if desired, using filtration, centrifugation and various chromatographic methods such as HPLC or affinity chromatography. Fragments of the monoclonal antibodies of the disclosure can be obtained from the purified monoclonal antibodies by methods that include digestion with enzymes, such as pepsin or papain, and/or by cleavage of disulfide bonds by chemical reduction. Alternatively, monoclonal antibody fragments encompassed by the present disclosure can be synthesized using an automated peptide synthesizer.

It also is contemplated that a molecular cloning approach may be used to generate monoclonals. For this, combinatorial immunoglobulin phagemid libraries are prepared from RNA isolated from the spleen of the immunized animal, and phagemids expressing appropriate antibodies are selected by panning using cells expressing the antigen and control cells e.g., normal-versus-tumor cells. The advantages of this approach over conventional hybridoma techniques are that approximately 10⁴ times as many antibodies can be produced and screened in a single round, and that new specificities are generated by H and L chain combination which further increases the chance of finding appropriate antibodies.

### C. Immunoassays

The present disclosure thus concerns immunodetection methods for binding, quantifying or otherwise generally detecting polyPs. The steps of various useful immunodetection methods have been described in the scientific literature, such as, *e.g.,* Nakamura *et al.* (1987). Immunoassays, in their most simple and direct sense, are binding assays. Certain immunoassays are the various types of enzyme linked immunosorbent assays (ELISAs), radioimmunoassays (RIA) and immunobead capture assay. However, it will be readily appreciated that detection is not limited to such techniques, and Western blotting, dot blotting, and the like also may be used in connection with the present disclosure.

In general, immunobinding methods include obtaining a sample suspected of containing a polyP, or fragment, and contacting the sample (such as blood, serum or plasma) with an antibody or protein or peptide in accordance with the present disclosure, as the case may be, under conditions effective to allow the formation of immunocomplexes.

The immunobinding methods of this disclosure include methods for detecting or quantifying the amount of a reactive component in a sample, which methods require the detection or quantitation of any immune complexes formed during the binding process. Here, one would obtain a sample suspected of containing extracellular polyP, and contact the sample with an antibody, and then detect or quantify the amount of immune complexes formed under the specific conditions.

Contacting the chosen biological sample with the antibody or antisera under conditions effective and for a period of time sufficient to allow the formation of immune complexes (primary immune complexes) is generally a matter of simply adding the composition to the sample and incubating the mixture for a period of time long enough for the antibodies to form immune complexes with extracellular polyPs. After this time, the sample-antibody composition will generally be washed to remove any non-specifically bound antibody species, allowing only those antibodies specifically bound within the primary immune complexes to be detected.

In general, the detection of immunocomplex formation is well known in the art and may be achieved through the application of numerous approaches. These methods are generally based upon the detection of a label or marker, such as any radioactive, fluorescent, biological or enzymatic tags or labels of standard use in the art. U.S. patents concerning the use of such labels include U.S. Pat. No. 3,817,837; U.S. Pat. No. 3,850,752; U.S. Pat. No. 3,939,350; U.S. Pat. No. 3,996,345; U.S. Pat. No. 4,277,437; U.S. Pat. No. 4,275,149 and U.S. Pat. No. 4,366,241.

In certain embodiments, the first added component that becomes bound within the primary immune complexes may be detected by means of a second binding ligand that has binding affinity for the encoded protein, peptide or corresponding antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under conditions effective and for a period of time sufficient to allow the formation of secondary immune complexes. The secondary immune complexes are then generally washed to remove any non-specifically bound labeled secondary antibodies or ligands, and the remaining label in the secondary immune complexes is then detected.

Further methods include the detection of primary immune complexes by a two step approach. A second binding ligand, such as an antibody, that has binding affinity for the encoded protein, peptide or corresponding antibody is used to form secondary immune complexes, as described above. After washing, the secondary immune complexes are contacted with a third binding ligand or antibody that has binding affinity for the second antibody, again under conditions effective and for a period of time sufficient to allow the formation of immune complexes (tertiary immune complexes). The third ligand or antibody is linked to a detectable label, allowing detection of the tertiary immune complexes thus formed. This system may provide for signal amplification if this is desired.

Of particular interest in the present disclosure are enzyme linked immunosorbent assays, known as ELISAs. In one exemplary ELISA, antibodies binding to the encoded proteins of the disclosure are immobilized directly in a well in a polystyrene microtiter plate. Then, a test composition suspected of containing the extracellular polyPs is added to the wells. After binding and washing to remove non-specifically bound immunocomplexes, the bound antigen may be detected.

Detection is generally achieved by the addition of a second antibody specific for the target antigen or other detection method, which is linked to a detectable label. This type of ELISA is a simple "sandwich ELISA." Detection also may be achieved by the addition of a biotinylated second antibody against polyP, followed by the addition of avidin or streptavidin that has been linked to a detectable label.

In another exemplary ELISA, the samples suspected of containing the extracellular polyPs are immobilized onto the well surface and then contacted with the antibodies of the disclosure. After binding and washing to remove non-specifically bound immune complexes, the bound antibody is detected. Where the initial antibodies are linked to a detectable label, the immune complexes may be detected directly. Again, the immune complexes may be detected using a second antibody that has binding affinity for the first antibody, with the second antibody being linked to a detectable label.

Another ELISA in which the extracellular polyPs are immobilized, involves the use of antibody competition in the detection. In this ELISA, labeled antibodies are added to the wells, allowed to bind to the extracellular polyPs and detected by means of their label. The amount of marker antigen in an unknown sample is then determined by mixing the sample with the labeled antibodies before or during incubation with coated wells. The presence of marker antigen in the sample acts to reduce the amount of antibody available for binding to the well and thus reduces the ultimate signal. This is appropriate for detecting antibodies in an unknown sample, where the unlabeled antibodies bind to the antigen-coated wells and also reduces the amount of antigen available to bind the labeled antibodies.

Irrespective of the format employed, ELISAs have certain features in common, such as coating, incubating or binding, washing to remove non-specifically bound species, and detecting the bound immune complexes. For example, in coating a plate with either antigen or antibody, one will generally incubate the wells of the plate with a solution of the antigen or antibody, either overnight or for a specified period of hours. The wells of the plate will then be washed to remove incompletely adsorbed material. Any remaining available surfaces of the wells are then "coated" with a nonspecific protein that is antigenically neutral with regard to the test antisera. These include bovine serum albumin (BSA), casein and solutions of milk powder. The coating allows for blocking of nonspecific adsorption sites on the immobilizing surface and thus reduces the background caused by nonspecific binding of antisera onto the surface.

In ELISAs, it is more customary to use a secondary or tertiary detection means rather than a direct procedure. Thus, after binding of a protein or antibody to the well, coating with a non-reactive material to reduce background, and washing to remove unbound material, the immobilizing surface is contacted with a control and sample to be tested under conditions effective to allow immune complex (antigen/antibody) formation. Detection of the immune complex then requires a labeled secondary binding ligand or antibody, or a secondary binding ligand or antibody in conjunction with a labeled tertiary antibody or third binding ligand.

"Under conditions effective to allow immune complex (antigen/antibody) formation" means that the conditions preferably include diluting the antigens and antibodies with solutions such as BSA, bovine gamma globulin (BGG) and phosphate buffered saline (PBS)/Tween. These added agents also tend to assist in the reduction of nonspecific background.

The "suitable" conditions also mean that the incubation is at a temperature and for a period of time sufficient to allow effective binding. Incubation steps are typically from about 1 to 2 to 4 hrs, at temperatures preferably on the order of 25.degree. C. to 27.degree. C., or may be overnight at about 4.degree. C. or so.

Following all incubation steps in an ELISA, the contacted surface is washed so as to remove non-complexed material. A washing procedure includes washing with a solution such as PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. Preferably, this will be an enzyme that will generate color development upon incubating with an appropriate chromogenic substrate. Thus, for example, one will desire to contact and incubate the first or second immune complex with a urease, glucose oxidase, alkaline phosphatase or hydrogen peroxidase-conjugated antibody for a period of time and under conditions that favor the development of further immune complex formation (e.g., incubation for 2 hrs at room temperature in a PBS-containing solution such as PBS-Tween).

After incubation with the labeled antibody, and subsequent to washing to remove unbound material, the amount of label is quantified, e.g., by incubation with a chromogenic substrate such as urea and bromocresol purple or 2,2'-azido-di-3-ethyl-benzthiazoline-6-sulfonic acid (ABTS) and H₂O₂, in the case of peroxidase as the enzyme label. Quantitation is then achieved by measuring the degree of color generation, e.g., using a visible spectra spectrophotometer.

### IV. Diagnosis and Therapy

In certain aspects, the present disclosure relates to the diagnosis and treatment of disorders that have as a component the production of toxic amounts of extracellular polyPs. By using agents that bind and block the function of the extracellular polyPs, the inventors seek to reduce and inhibit the toxic effects of these molecules. In addition, the presence of such extracellular polyPs, alone or in conjunction with other diagnostic features, may identify subjects at risk of developing life-threatening hyper-inflammatory or hyper-coagulation reactions. Thus, assays to detect extracellular polyPs in samples, such as blood, plasma and serum, also are proposed.

### A. Diagnosis/Prognosis

In one aspect, the present disclosure will entail obtaining a biological sample from a patient at risk of or suspected of having a hyper-inflammatory or hyper-coagulation condition involving extracellular polyP production and toxicity. Biological samples will typically entail blood, plasma or serum, but other fluids such as saliva, sputum, cerebrospinal fluid, lung lavage, and urine may be utilized. Employing the immunological assays described above, or other techniques (e.g., mass spectrometry such as MALDI-TOF), the polyP content of the sample is assessed, with elevated levels of polyPs being indicative of a hyper-inflammatory or hyper-coagulation disorder. The subject may then be treated, as discussed below, or simply monitored for further progression or recovery.

### B. Therapies

The present disclosure contemplates the use of inhibitors of extracellular polyP toxicity to treat a variety of hyper-inflammatory disease or hyper-coagulation disease or other toxic states specified above. The inventors contemplate the use of antibodies to polyPs. Also contemplated are mixtures of agents, including at least one anti-polyP antibody with at least one anti-histone antibody.

The therapies may include mAbs at least one anti-polyP antibody that may be humanized by any suitable method, as are known to those of skill in the art, for therapeutic administration to humans. The therapies may include mAbs at least one anti-polyP antibody that are not humanized for therapeutic administration to humans.

The therapies may include mAbs at least one anti-histone antibody that may be humanized by any suitable method, as are known to those of skill in the art, for therapeutic administration to humans. The therapies may include mAbs at least one anti-histone antibody that are not humanized for therapeutic administration to humans.

Treatment regimens will vary depending on the severity and type of disease, the overall health and age of the patient, and various other conditions to be taken into account by the treating physician. Multiple doses or treatments may be applied, as well as "continuous" therapy where a small amount of the therapeutic agent is provided continually over an extended period of time. The agent may also be provided in a single bolus administration, but is formulated to provide delayed, timed or extended release of the active form.

In addition, combinations of an inhibitor of extracellular polyP toxicity with other treatments may be used by administration of a single composition or pharmacological formulation that includes both multiple agents, or by administering two distinct compositions or formulations, at the same time. Alternatively, one treatment may precede or follow administration of the other by intervals ranging from minutes to weeks. In embodiments where the two agents are applied separately, one would generally ensure that a significant period of time did not expire between the time of each delivery, such that both agents would still be able to exert an advantageously combined effect. In such instances, it is contemplated that one would typically administer both modalities within about 12-24 hours of each other and, more preferably, within about 6-12 hours of each other, with a delay time of only about 12 hours may be used. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations. It also is conceivable that more than one administration of a drug will be desired.

By way of illustration, the following permutations based on 3 and 4 total administrations are exemplary, where A represents a first inhibitor of extracellular polyP toxicity and B represents a second drug (including a second inhibitor of extracellular polyP toxicity):
A/B/A B/A/B B/B/A A/A/B B/A/A A/B/B B/B/B/A B/B/A/B A/A/B/B A/B/A/B A/B/B/A B/B/A/A B/A/B/A B/A/A/B B/B/B/A A/A/A/B B/A/A/A A/B/A/A A/A/B/A A/B/B/B B/A/B/B B/B/A/B
Other combinations are likewise contemplated.

### C. Pharmaceutical Formulations and Routes of Administration

Where clinical applications are contemplated, pharmaceutical compositions including polyP, fragments and anti-polyP antibodies, and mixtures thereof, will be prepared in a form appropriate for the intended application. Generally, this will entail preparing compositions that are essentially free of pyrogens, as well as other impurities that could be harmful to humans or animals.

One will generally desire to employ appropriate salts and buffers to render delivery vectors stable and allow for uptake by target cells. Buffers also will be employed when recombinant cells are introduced into a patient. Aqueous compositions of the present disclosure comprise an effective amount of the vector or cells, dissolved or dispersed in a pharmaceutically acceptable carrier or aqueous medium. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes solvents, buffers, solutions, dispersion media, coatings, antibacterial and antifungal agents, isotonic and absorption delaying agents and the like acceptable for use in formulating pharmaceuticals, such as pharmaceuticals suitable for administration to humans. The use of such media and agents for pharmaceutically active substances is well known in the art. Except insofar as any conventional media or agent is incompatible with the active ingredients of the present disclosure, its use in therapeutic compositions is contemplated. Supplementary active ingredients also can be incorporated into the compositions, provided they do not inactivate the vectors or cells of the compositions.

The active compositions of the present disclosure may include classic pharmaceutical preparations. Administration of these compositions according to the present disclosure may be via any common route so long as the target tissue is available via that route. This includes oral, nasal, or buccal. Alternatively, administration may be by intradermal, subcutaneous, intramuscular, intraperitoneal or intravenous injection. Such compositions would normally be administered as pharmaceutically acceptable compositions, as described supra.

The active compounds may also be administered parenterally or intraperitoneally. By way of illustration, solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations generally contain a preservative to prevent the growth of microorganisms.

The pharmaceutical forms suitable for injectable use include, for example, sterile aqueous solutions or dispersions and sterile powders for the extemporaneous preparation of sterile injectable solutions or dispersions. Generally, these preparations are sterile and fluid to the extent that easy injectability exists. Preparations should be stable under the conditions of manufacture and storage and should be preserved against the contaminating action of microorganisms, such as bacteria and fungi. Appropriate solvents or dispersion media may contain, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions may be prepared by incorporating the active compounds in an appropriate amount into a solvent along with any other ingredients (for example as enumerated above) as desired, followed by filtered sterilization. Generally, dispersions are prepared by incorporating the various sterilized active ingredients into a sterile vehicle which contains the basic dispersion medium and the desired other ingredients, e.g., as enumerated above. In the case of sterile powders for the preparation of sterile injectable solutions, the methods of preparation include vacuum-drying and freeze-drying techniques which yield a powder of the active ingredient(s) plus any additional desired ingredient from a previously sterile-filtered solution thereof.

The compositions of the present disclosure generally may be formulated in a neutral or salt form. Pharmaceutically-acceptable salts include, for example, acid addition salts (formed with the free amino groups of the protein) derived from inorganic acids (*e.g*., hydrochloric or phosphoric acids, or from organic acids (*e.g*., acetic, oxalic, tartaric, mandelic, and the like. Salts formed with the free carboxyl groups of the protein can also be derived from inorganic bases (*e.g*., sodium, potassium, ammonium, calcium, or ferric hydroxides) or from organic bases (*e.g*., isopropylamine, trimethylamine, histidine, procaine and the like.

Upon formulation, solutions are preferably administered in a manner compatible with the dosage formulation and in such amount as is therapeutically effective. The formulations may easily be administered in a variety of dosage forms such as injectable solutions, drug release capsules and the like. For parenteral administration in an aqueous solution, for example, the solution generally is suitably buffered and the liquid diluent first rendered isotonic for example with sufficient saline or glucose. Such aqueous solutions may be used, for example, for intravenous, intraarterial, intramuscular, subcutaneous and intraperitoneal administration. Preferably, sterile aqueous media are employed as is known to those of skill in the art, particularly in light of the present disclosure. By way of illustration, a single dose may be dissolved in 1 ml of isotonic NaCl solution and either added to 1000 ml of hypodermoclysis fluid injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### V. Examples

The following example is included to demonstrate embodiments of the disclosure.

### Example 1

Autoimmune mice (NZBWF1/J) generate antibodies against a variety of their own proteins, often intracellular proteins. The auto immune mice were screened to determine if any mice were producing antibodies to polyP. PolyP for the assay was biotinylated and bound to the screening plate via streptavidin. Antibodies that bound to the polyP were then detected by a standard enzyme linked immune assay (ELISA) using biotinylated polyP captured on 96-well plates that had been coated with streptavidin.

Antibodies were detected and developed as monoclonal antibodies using standard technique. A particular anti-polyP monoclonal antibody was named PP2055. However, other anti-polyP antibodies are contemplated from the auto immune mice, and other antibody producing sources.

As shown in FIG. 2, in coagulation assays in which coagulation was initiated by recalcification of citrated human plasma in the presence of polyP, the polyP shortened the clotting time relative to control without polyP. Addition of PP2055 in the presence of polyP neutralized most of the polyP clot promoting activity, when the anti-polyP antibody concentration was high enough. On the other hand, rat IgG did not significantly neutralize the polyP clot promoting activity, and neither did a lower concentration of the anti-polyP antibody. The mice were used according to an animal protocol approved by Institutional Animal Care and Use Committees of the Oklahoma Medical Research Foundation.

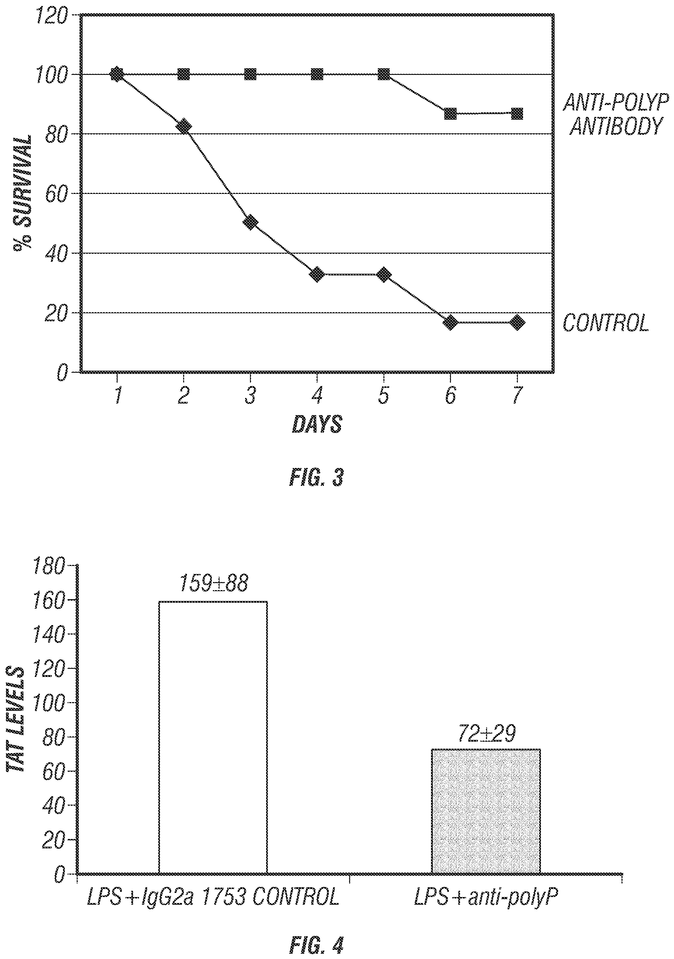

As shown in FIG. 3, neutralizing antibodies against polyP protected against death of the mice in the acute inflammatory model. *In vivo,* lipopolysaccharide (LPS) induces coagulation and can induce death. 10 mg/kg of LPS L6511 was administered to C57BL6/J mice by retroorbital injection. Response of the mice to the administration was examined. Most of the LPS-administered mice died, while most of the mice administered LPS plus PP2055 lived, which is a significant difference in outcome for the 2 mouse populations.

As shown in FIG. 4, PP2055 decreases coagulation in mice. As a surrogate for coagulation, the thrombin-antithrombin complex (TAT) levels in the mouse plasma were determined using the TAT kit from Siemens. Blood was taken by heart puncture 4 hours after LPS administration. The administration of PP2055 decreased the TAT levels approximately 50% suggesting that polyP is a major contributor to the coagulation induced by LPS, which is a significant difference.

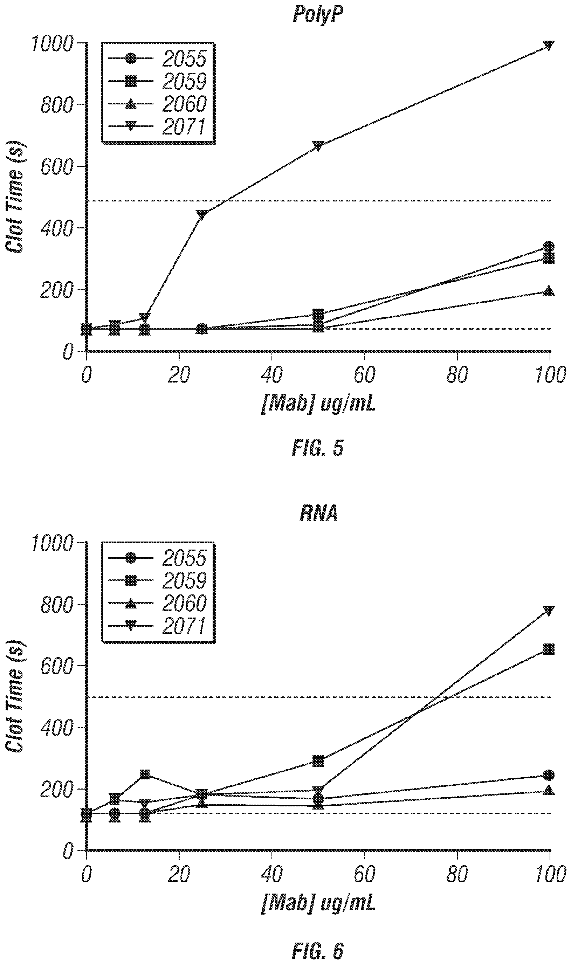

FIG. 5 shows the results of coagulation assays in which coagulation was initiated by recalcification of citrated human plasma in the presence of polyP. The addition of polyP shortened the clotting time relative to control without polyP, which is indicated by a horizontal dotted line. Addition of anti-polyP antibodies 2055, 2059, 2060 or 2071 all caused a prolongation of the clotting time in a dose-dependent manner, with antibody 2071 showing the greatest ability to block the clotting activity of polyP.

FIG. 6 shows the results of coagulation assays in which coagulation was initiated by recalcification of citrated human plasma in the presence of RNA (polyguanylic acid). The addition of RNA shortened the clotting time relative to control without RNA, which is indicated by a horizontal dotted line. Addition of anti-polyP antibodies 2059 or 2071 caused a prolongation of the clotting time in a dose-dependent manner.

FIG. 7 shows the results of plasma coagulation assays in which coagulation was initiated by either long-chain polyP (left panel) or RNA (right panel). The RNA used in this experiment was polyguanylic acid. Adding polyP or RNA to plasma shortened the clotting time relative to that observed with no added activator, as indicated by the horizontal dotted lines. The addition of anti-polyP antibody 2059 caused a prolongation of the clotting time in a dose-dependent manner.

### VI. References

U.S. Pat. No. 3,817,837
U.S. Pat. No. 3,850,752
U.S. Pat. No. 3,939,350
U.S. Pat. No. 3,996,345
U.S. Pat. No. 4,196,265
U.S. Pat. No. 4,275,149
U.S. Pat. No. 4,277,437
U.S. Pat. No. 4,366,241
U.S. Pat. No. 4,554,101
U.S. Pat. No. 5,440,013
U.S. Pat. No. 5,446,128
U.S. Pat. No. 5,475,085
U.S. Pat. No. 5,618,914
U.S. Pat. No. 5,670,155
U.S. Pat. No. 5,672,681
U.S. Pat. No. 5,674,976
U.S. Pat. No. 5,710,245
U.S. Pat. No. 5,840,833
U.S. Pat. No. 5,859,184
U.S. Pat. No. 5,929,237
U.S. Pat. No. 8,716,218
Abakushin et al., Biochemistry (Mosc), 64:693-698, 1999. Antibodies: A Laboratory Manual, Cold Spring Harbor Laboratory, Cold Spring Harbor Press, Cold Spring Harbor, N.Y., 1988.
Baltch et al., J. Antimicrob. Chemother., 59(6):1177-81, 2007.
Barany and Merrifield, In: The Peptides, Gross and Meienhofer (Eds.), Academic Press, NY, 1-284, 1979.
Bernard et al., NE J. Med., 344:699-709, 2001. Brinkmann et al., Science, 303:1532-1535, 2004.
Campbell, In: Monoclonal Antibody Technology, Laboratory Techniques in Biochemistry and Molecular Biology, Burden and Von Knippenberg (Eds.), Elsevier, Amsterdam, 13:71-74; 75-83, 1984.
Capaldi et al., Biochem. Biophys. Res. Comm., 74(2):425-433, 1977.
Choi et al., Blood, 118(26):6963-6970, 2011.
Clark et al., Nat. Med. 13, 463-469, 2007.
Currie et al., Biochim. Biophys. Acta, 1355:248-258, 1997.
Dinarvand et al., Blood, 123(6):935-945.
Emlen et al., J. Immunol., 148:3042-3048, 1992.
Esmon et al., J. Autoimmun., 15:221-225, 2000.
Esmon, Chest, 124:26 S-32S, 2003.
Gaini et al., Crit. Care, 11:R32, 2007.
Gefter et al., Somatic Cell Genet, 3:231-236, 1977.
Goding, In: Monoclonal Antibodies: Principles and Practice, 2d ed., Academic Press, Orlando, Fla., 60-66, and 71-74, 1986.
Gupta et al., J. Am. Soc. Nephrol., 18:860-867, 2007.
Herren et al., Biochemistry, 45:9463-9474, 2006.
Hirsch, J. Exp. Med., 108:925-944, 1958.
Johannesson et al., J. Med. Chem., 42(22):4524-4537, 1999.
Johnson et al., In: Biotechnology and Pharmacy, Pezzuto et al. (Eds.), Chapman and Hall, NY, 1993.
Kalaaji et al., Kidney Int., 71:664-672, 2007.
Kleine et al., Am. J. Physiol., 273:C1925-C1936, 1997.
Kohler and Milstein, Eur. J. Immunol., 6(7):511-519, 1976.
Kohler and Milstein, Nature, 256(5517):495-497, 1975.
Kyte and Doolittle, J. Mol. Biol., 157(1):105-132, 1982.
Lay et al., Blood, 109, 1984-1991:2007.
Macias & Nelson, Crit. Care Med. 32:S223-S228, 2004. Merrifield, Science, 232(4748):341-347, 1986.
Monestier et al., Mol. Immunol., 30:1069-1075, 1993.
Morrissey et al., Blood, 119(25):5972-5979, 2012.
Mosnier, Blood, 123(6):802-806, 2014.
Müller et al., Cell, 139(6):1143-1156, December 11, 2009.
Nakamura et al., In: Handbook of Experimental Immunology (4.sup.th Ed.), Weir (Eds), 1:27, Blackwell Scientific Publ., Oxford, 1987.
Radic et al., J. Immunol., 172:6692-6700, 2004.
Remington's Pharmaceutical Sciences, 15.sup.th Ed., 1035-1038 and 1570-1580, 1990. Riewald et al., Science 296, 1880-1882:2002.
Rouhiainen et al., J. Leukoc. Biol., 81:49-58, 2007.
Russell, N. Engl. J. Med., 355:1699-1713, 2006.
Semeraro et al.,Blood, 118(7):1952-61, 2011.
Smirnov & Esmon, J. Biol. Chem. 269:816-819, 1994.
Smith et al., J Thromb Haemost., 6(10):1750-6, 2008.
Smith et al., Blood, 112(7):2810-6, 2008.
Smith et al., Blood, 116(20):4353-4359, 2010. Erratum in Blood. 2011 Mar 24;117(12):3477.
Smith et al., Blood, 120(26):5103-5110, 2012
Stewart and Young, In: Solid Phase Peptide Synthesis, 2d. ed., Pierce Chemical Co., 1984.
Tam et al., J. Am. Chem. Soc., 105:6442, 1983.
Taylor et al., J. Clin. Invest., 79:918-925, 1987.
Wang et al., Science, 285:248-251, 1999.
Weisshoff et al., Eur. J. Biochem., 259(3):776-788, 1999.

## Claims

1. An anti-polyphosphate antibody for use in a method of treatment of a medical condition involving extracellular polyphosphate toxicity in a subject, the method comprising administering to said subject an anti-polyphosphate antibody.

2. The anti-polyphosphate antibody for use of claim 1, wherein said medical condition comprises hyper-inflammation and/or comprises hyper-coagulation.

3. The anti-polyphosphate antibody for use of claim 1, wherein said anti-polyphosphate antibody reduces disseminated intravascular coagulation in sepsis, minimizes post-surgical thrombosis, decreases lung injury in acute respiratory distress syndrome, reduces both coagulation and inflammatory pathway responses, thereby decreasing inflammation and edema due to bradykinin generation associated with the medical condition, or inhibits pro-inflammatory cytokine production by endothelial cells in said subject, in particular wherein the pro-inflammatory cytokine is IL-6 or IL-8.

4. The anti-polyphosphate antibody for use of claim 1, the method further comprising:
i) administering to said subject an anti-histone antibody to inhibit said medical condition involving extracellular polyphosphate toxicity in the subject; or
ii) determining an extracellular polyphosphate content in a serum or plasma sample from said subject; and
iii)adjusting a dosage of said administered anti-polyphosphate antibody responsive to said extracellular polyphosphate content.

5. A method of assessing a disease state of a subject comprising:
determining the extracellular polyphosphate content of a serum or plasma sample obtained from said subject previously, wherein a measurement of extracellular polyphosphate in said sample using an anti-polyphosphate antibody provides a diagnosis of the disease state, a prognosis of the disease state or indicates a treatment response of the disease state.

6. The method of claim 5, wherein the measurement of the extracellular polyphosphate provides a diagnosis of the disease state, and the diagnosis is used to determine the treatment for said subject or wherein the measurement of the extracellular polyphosphate provides a prognosis of the disease state, and the prognosis is used to determine the treatment for said subject, or wherein the measurement of the extracellular polyphosphate indicates a treatment response of the disease state, and the treatment response is used to determine further treatment for said subject.

7. The method of claim 5, wherein the determining step comprises ELISA or Western blotting using said anti-polyphosphate antibodies.

8. An anti-polyphosphate antibody for use in a method of treating a disease state in a subject, wherein the method comprises:
obtaining a serum or plasma sample from said subject;
determining the extracellular polyphosphate content of said sample, wherein a measurement of extracellular polyphosphate in said sample indicates existence or status of the disease state of the subject, and
further comprising treating said subject with said anti-polyphosphate antibody.

9. A medical device configured for treating a medical condition involving extracellular polyphosphate toxicity in a subject comprising neutralizing polyphosphate during extracorporeal blood flow from the subject, such that the blood flow returned to the subject has reduced extracellular polyphosphate toxicity, wherein the medical device comprises an anti-polyphosphate antibody, and wherein neutralizing polyphosphate comprises binding of polyphosphate to said anti-polyphosphate antibody.

10. The medical device of claim 9, wherein the extracorporeal blood flow is through a cardiac bypass machine used during cardiac bypass surgery or wherein the extra corporeal blood flow is via extracorporeal life-support configured for cardiac and/or respiratory support that allows the heart and/or lungs of the subject to rest and recover.

11. The method of claim 1 or 5 or the medical device of claim 9, wherein said subject is selected from a group consisting of human, dog, cat, horse, monkey, mouse, rat, rabbit, sheep, goat, cow, and pig.

12. The medical device of claim 9, wherein said medical condition is selected from the group consisting of bacterial sepsis, anthrax, virus, Ebola, fungal sepsis, hemorrhagic fevers, surgery, traumatic hemorrhage and/or tissue damage, edema, trauma, acute pancreatitis, acute respiratory distress syndrome, ischemia-reperfusion injury, vascular leak, circulatory shock, cancer, cardiovascular disease, autoimmune disease, chemotherapy toxicity, radiotherapy toxicity, cytokine therapy toxicity and burn.

13. The anti-polyphosphate antibody for use according to claim 1, the method comprising administering to said subject the anti-polyphosphate antibody *in vivo,* wherein said anti-polyphosphate antibody cross-reacts with a ribonucleic acid or deoxyribonucleic acid.

14. The anti-polyphosphate antibody for use of claim 1 or claim 13, wherein said medical condition is selected from the group consisting of bacterial sepsis, anthrax, virus, Ebola, fungal sepsis, hemorrhagic fevers, surgery, traumatic hemorrhage and/or tissue damage, edema, trauma, acute pancreatitis, acute respiratory distress syndrome, ischemia-reperfusion injury, vascular leak, circulatory shock, cancer, cardiovascular disease, autoimmune disease, chemotherapy toxicity, radiotherapy toxicity, cytokine therapy toxicity and burn.

## Patentansprüche

1. Ein Anti-Polyphosphat-Antikörper zur Verwendung in einem Verfahren zur Behandlung eines medizinischen Zustands, der mit extrazellulärer Polyphosphat-Toxizität in einer Person verbunden ist, wobei das Verfahren Verabreichen eines Anti-Polyphosphat-Antikörpers an besagte Person umfasst.

2. Der Anti-Polyphosphat-Antikörper zur Verwendung nach Anspruch 1, wobei besagter medizinischer Zustand Hyperinflammation und/oder Hyperkoagulation umfasst.

3. Der Anti-Polyphosphat-Antikörper zur Verwendung nach Anspruch 1, wobei besagter Anti-Polyphosphat-Antikörper disseminierte intravasale Koagulation bei Sepsis reduziert, postoperative Thrombose minimiert, Lungenschädigung bei akutem Lungenversagen verringert, sowohl Koagulations- als auch Entzündungssignalweg-Reaktionen reduziert, wobei dadurch eine Entzündung und ein Ödem aufgrund einer mit dem medizinischen Zustand assoziierten Bradykininerzeugung verringert wird, oder eine proinflammatorische Cytokinproduktion von Endothelzellen in besagter Person hemmt, insbesondere wobei das proinflammatorische Cytokin IL-6 oder IL-8 ist.

4. Der Anti-Polyphosphat-Antikörper zur Verwendung nach Anspruch 1, wobei das Verfahren außerdem umfasst:
i) Verabreichen eines Anti-Histon-Antikörpers an besagte Person, um besagten medizinischen Zustand zu hemmen, der mit extrazellulärer Polyphosphat-Toxizität in der Person verbunden ist; oder
ii) Bestimmen eines extrazellulären Polyphosphatgehalts in einer Serum- oder Plasmaprobe von besagter Person; und
iii) Anpassen einer Dosis besagten verabreichten Anti-Polyphosphat-Antikörpers, der auf besagten extrazellulären Polyphosphatgehalt reagiert.

5. Ein Verfahren zur Bewertung eines Krankheitsstadiums einer Person umfassend:
Bestimmen des extrazellulären Polyphosphatgehalts einer von besagter Person zuvor entnommenen Serum- oder Plasmaprobe, wobei eine Messung extrazellulären Polyphosphats in besagter Probe unter Verwendung eines Anti-Polyphosphat-Antikörpers eine Diagnose des Krankheitsstadiums oder eine Prognose des Krankheitsstadiums bereitstellt oder einen Behandlungserfolg des Krankheitsstadiums anzeigt.

6. Das Verfahren nach Anspruch 5, wobei die Messung des extrazellulären Polyphosphats eine Diagnose des Krankheitsstadiums bereitstellt und die Diagnose zur Bestimmung der Behandlung für besagte Person verwendet wird oder wobei die Messung des extrazellulären Polyphosphats eine Prognose des Krankheitsstadiums bereitstellt und die Prognose zur Bestimmung der Behandlung für besagte Person verwendet wird oder wobei die Messung des extrazellulären Polyphosphats einen Behandlungserfolg des Krankheitsstadiums anzeigt und der Behandlungserfolg zur Bestimmung einer weiteren Behandlung für besagte Person genutzt wird.

7. Das Verfahren nach Anspruch 5, wobei der Bestimmungsschritt ELISA oder Western Blotting unter Verwendung besagter Anti-Polyphosphat-Antikörper umfasst.

8. Ein Anti-Polyphosphat-Antikörper zur Verwendung in einem Verfahren zur Behandlung eines Krankheitsstadiums in einer Person, wobei das Verfahren umfasst:
Entnahme einer Serum- oder Plasmaprobe von besagter Person;
Bestimmen des extrazellulären Polyphosphatgehalts besagter Probe, wobei eine Messung extrazellulären Polyphosphats in besagter Probe ein Vorliegen oder ein Status des Krankheitsstadiums der Person anzeigt, und
außerdem umfassend Behandlung besagter Person mit besagtem Anti-Polyphosphat-Antikörper.

9. Eine medizinische Vorrichtung, die zur Behandlung eines medizinischen Zustands konfiguriert ist, der mit extrazellulärer Polyphosphat-Toxizität in einer Person verbunden ist, umfassend Neutralisieren von Polyphosphat während eines extrakorporalen Blutstroms aus der Person, so dass der Blutstrom, welcher der Person rückgeführt wird, eine reduzierte extrazelluläre Polyphosphat-Toxizität besitzt, wobei die medizinische Vorrichtung einen Anti-Polyphosphat-Antikörper umfasst und wobei Neutralisieren von Polyphosphat ein Binden von Polyphosphat an besagten Anti-Polyphosphat-Antikörper umfasst.

10. Die medizinische Vorrichtung nach Anspruch 9, wobei der extrakorporale Blutstrom durch eine Herz-Bypass-Maschine geführt wird, die während einer Herz-Bypass-Operation eingesetzt wird, oder wobei der extrakorporale Blutstrom mithilfe einer extrakorporalen Lebenserhaltung erfolgt, die für eine Herz- und/oder Atmungsunterstützung konfiguriert ist, die dem Herz und/oder den Lungen der Person eine Schonung und Regeneration erlaubt.

11. Das Verfahren nach Anspruch 1 bis 5 oder die medizinische Vorrichtung nach Anspruch 9, wobei besagte Person aus der Gruppe ausgewählt ist, bestehend aus Mensch, Hund, Katze, Pferd, Affe, Maus, Ratte, Kaninchen, Schaf, Ziege, Kuh und Schwein.

12. Die medizinische Vorrichtung nach Anspruch 9, wobei besagter medizinischer Zustand aus der Gruppe ausgewählt ist, bestehend aus bakterieller Sepsis, Anthrax, Viren, Ebola, fungaler Sepsis, hämorrhagischen Fiebern, Operation, traumatischer Hämorrhagie und/oder Gewebeschädigung, Ödem, Trauma, akuter Pankreatitis, akutem Lungenversagen, ischämischen Reperfusionsschäden, Gefäßlekage, Kreislaufschock, Krebs, Herz-Kreislauf-Erkrankung, Autoimmunkrankheit, chemotherapeutischer Toxizität, strahlentherapeutischer Toxizität, Cytokintherapie-Toxizität und Verbrennungen.

13. Der Anti-Polyphosphat-Antikörper zur Verwendung gemäß Anspruch 1, wobei das Verfahren Verabreichen des Anti-Polyphosphat-Antikörpers an die Person *in vivo* umfasst, wobei besagter Anti-Polyphosphat-Antikörper mit einer Ribonukleinsäure oder Desoxyribonukleinsäure kreuzreagiert.

14. Der Anti-Polyphosphat-Antikörper zur Verwendung gemäß Anspruch 1 oder Anspruch 13, wobei besagter medizinischer Zustand aus der Gruppe ausgewählt ist, bestehend aus bakterieller Sepsis, Anthrax, Viren, Ebola, fungaler Sepsis, hämorrhagischen Fiebern, Operation, traumatischer Hämorrhagie und/oder Gewebeschädigung, Ödem, Trauma, akuter Pankreatitis, akutem Lungenversagen, ischämischen Reperfusionsschäden, Gefäßlekage, Kreislaufschock, Krebs, Herz-Kreislauf-Erkrankung, Autoimmunkrankheit, chemotherapeutischer Toxizität, strahlentherapeutischer Toxizität, Cytokintherapie-Toxizität und Verbrennungen.

## Revendications

1. Anticorps anti-polyphosphate pour une utilisation dans une méthode de traitement d'un état médical impliquant une toxicité au polyphosphate extracellulaire chez un sujet, la méthode comprenant l'administration audit sujet d'un anticorps anti-polyphosphate.

2. Anticorps anti-polyphosphate pour une utilisation selon la revendication 1, dans lequel ledit état médical comprend une hyper-inflammation et/ou comprend une hyper-coagulation.

3. Anticorps anti-polyphosphate pour une utilisation selon la revendication 1, lequel anticorps anti-polyphosphate réduit la coagulation intravasculaire disséminée lors d'une sepsie, minimise une thrombose post-chirurgicale, diminue les lésions pulmonaires lors d'un syndrome de détresse respiratoire aiguë, réduit les réponses aux voies tant de coagulation que d'inflammation, en diminuant ainsi l'inflammation et l'oedème dus à la génération de bradykinine associée à l'état médical, ou inhibe la production de cytokines pro-inflammatoires par les cellules endothéliales chez ledit sujet, en particulier dans lequel la cytokine pro-inflammatoire est IL-6 ou IL-8.

4. Anticorps anti-polyphosphate pour une utilisation selon la revendication 1, dans lequel la méthode comprend en outre :
i) l'administration audit sujet d'un anticorps anti-histone pour inhiber ledit état médical impliquant une toxicité au polyphosphate extracellulaire chez le sujet ; ou
ii) la détermination de la teneur en polyphosphate extracellulaire dans un échantillon de sérum ou de plasma provenant dudit sujet ; et
iii) l'ajustement de la dose dudit anticorps anti-polyphosphate administré en réponse à ladite teneur en polyphosphate extracellulaire.

5. Méthode de détermination d'un état pathologique chez un sujet, comprenant :
la détermination de la teneur en phosphate extracellulaire d'un échantillon de sérum ou de plasma obtenu antérieurement auprès dudit sujet, où la mesure du polyphosphate extracellulaire dans ledit échantillon, utilisant un anticorps anti-polyphosphate, donne un diagnostic de l'état pathologique, un pronostic de l'état pathologique, ou indique une réponse au traitement de l'état pathologique.

6. Méthode selon la revendication 5, dans laquelle la mesure du polyphosphate extracellulaire donne un diagnostic de l'état pathologique, et le diagnostic est utilisé pour déterminer le traitement pour ledit sujet, ou dans laquelle la mesure du polyphosphate extracellulaire donne un pronostic de l'état pathologique, et le pronostic est utilisé pour déterminer le traitement pour ledit sujet, ou dans laquelle la mesure du polyphosphate extracellulaire indique une réponse au traitement de l'état pathologique, et la réponse au traitement est utilisée pour déterminer un traitement ultérieur pour ledit sujet.

7. Méthode selon la revendication 5, dans laquelle l'étape de détermination comprend un ELISA ou un transfert Western utilisant lesdits anticorps anti-polyphosphate.

8. Anticorps anti-polyphosphate pour une utilisation dans une méthode de traitement d'un état pathologique chez un sujet, dans lequel la méthode comprend :
l'obtention d'un échantillon de sérum ou de plasma auprès dudit sujet ;
la détermination de la teneur en polyphosphate extracellulaire dudit échantillon, où une mesure de polyphosphate extracellulaire dans ledit échantillon indique l'existence ou le statut de l'état pathologique du sujet, et
comprend en outre le traitement du sujet avec ledit anticorps anti-polyphosphate.

9. Dispositif médical configuré pour traiter un état médical impliquant une toxicité au polyphosphate extracellulaire chez un sujet, comprenant la neutralisation du polyphosphate durant une circulation sanguine extracorporelle du sujet, de façon que le flux sanguin renvoyé dans le sujet ait une toxicité au polyphosphate réduite, lequel dispositif médical comprend un anticorps anti-polyphosphate, dans lequel la neutralisation du polyphosphate comprend la liaison du polyphosphate audit anticorps anti-polyphosphate.

10. Dispositif médical selon la revendication 9, dans lequel la circulation sanguine extracorporelle a lieu par l'intermédiaire d'une machine de dérivation cardiaque utilisée durant une opération de dérivation cardiaque, ou dans lequel la circulation sanguine extracorporelle a lieu via un support de vie extracorporel configuré pour un support cardiaque et/ou respiratoire qui permet au coeur et/ou aux poumons du sujet de se reposer et de récupérer.

11. Méthode selon la revendication 1 ou 5 ou dispositif médical selon la revendication 9, dans lequel ledit sujet est choisi dans le groupe constitué par un humain, un chien, un chat, un cheval, un singe, une souris, un rat, un lapin, un mouton, une chèvre, une vache, et un cochon.

12. Dispositif médical selon la revendication 9, dans lequel ledit état médical est choisi dans le groupe constitué par une septicémie bactérienne, l'anthrax, un virus, Ebola, une septicémie fongique, une fièvre hémorragique, une opération chirurgicale, une hémorragie traumatique et/ou un dommage tissulaire, un oedème, un trauma, une pancréatite aiguë, un syndrome de détresse respiratoire aiguë, une lésion après ischémie-reperfusion, une fuite vasculaire, un choc circulatoire, un cancer, une maladie cardiovasculaire, une maladie auto-immune, une toxicité à une chimiothérapie, une toxicité à une radiothérapie, une toxicité à une thérapie par une cytokine, et une brûlure.

13. Anticorps anti-polyphosphate pour une utilisation selon la revendication 1, dans lequel la méthode comprend l'administration audit sujet de l'anticorps anti-polyphosphate in vivo, lequel anticorps anti-polyphosphate réagit de façon croisée avec un acide ribonucléique ou un acide désoxyribonucléique.

14. Anticorps anti-polyphosphate pour une utilisation selon la revendication 1 ou 13, dans lequel ledit état médical est choisi dans le groupe constitué par une septicémie bactérienne, l'anthrax, un virus, Ebola, une septicémie fongique, une fièvre hémorragique, une opération chirurgicale, une hémorragie traumatique et/ou un dommage tissulaire, un oedème, un trauma, une pancréatite aiguë, un syndrome de détresse respiratoire aiguë, une lésion après ischémie-reperfusion, une fuite vasculaire, un choc circulatoire, un cancer, une maladie cardiovasculaire, une maladie auto-immune, une toxicité à une chimiothérapie, une toxicité à une radiothérapie, une toxicité à une thérapie par une cytokine, et une brûlure.
